# EUROPÄISCHE PATENTSCHRIFT

(11) **EP 1 713 433 B1**
(45) Veröffentlichungstag und Bekanntmachung des Hinweises auf die Patenterteilung: **30.05.2007**
(21) Anmeldenummer: 05701408.6
(22) Anmeldetag: 10.02.2005
(51) Int. Cl.: A61K 8/18

(54) **ANTIFALTEN-KOSMETIKUM**
ANTI-WRINKLE COSMETIC
PRODUIT COSMETIQUE ANTIRIDES

(30) Priorität: 11.02.2004 DE 102004007385
(43) Veröffentlichungstag der Anmeldung: 25.10.2006
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC); DOUCET, Olivier, F-06230 Villefranche s/Mer (FR)
(74) Vertreter: Walter, Wolf-Jürgen
(86) Internationale Anmeldenummer: PCT/EP2005/001460
(87) Internationale Veröffentlichungsnummer: WO 2005/077328

(56) Entgegenhaltungen:
- EP-A- 1 112 079
- EP-A- 1 281 396
- FR-A- 2 661 090
- FR-A- 2 846 885
- DATABASE WPI Section Ch, Week 199331 Derwent Publications Ltd., London, GB; Class B04, AN 1993-245182 XP002324979 & ES 2 038 081 A1 (POZUELO HERGUIDO P) 1. Juli 1993 (1993-07-01)

## Beschreibung

Die Erfindung betrifft ein neues Antifalten-Kosmetikum auf Basis natürlicher Pflanzenextrakte.

Der Einsatz von Pflanzenextrakten in kosmetischen Mitteln ist bekannt. Überraschend ist oft bei Kombinationen mehrerer Pflanzenextrakte die daraus resultierende Gesamtwirkung, die nicht immer den Einzelwirkungen entspricht und auch von der Art der ausgewählten Pflanzenteile und der Extraktionsmittel abhängig ist. Von Mohn (Papaver) sind wegen des Alkaloidgehaltes meist nur innerliche Wirkungen bekannt. Auch Passiflora-Extrakte sind zumeist innerlich als krampflösendes oder entzündungshemmendes Mittel verwendet worden. Die EP 1002524 B1 beschreibt ein Gemisch von Muskatrosenöl, Kamelienöl und Sonnenblumenöl, das auch Passionsblumenöl enthalten kann, als Mittel mit Anti-Aging-Wirkung. Minze (Menthae) ist äußerlich bisher insbesondere bei Hautreizungen eingesetzt worden oder zusammen mit anderen Pflanzen zur Hautaufhellung (WO02/065999). Die WO 03/083028 beschreibt ein Körperreinigungsmittel, das Mentha spicata var. Viridis als Wirkstoff enthält.

Der Erfindung liegt die Aufgabe zugrunde, ein Kosmetikum zur Faltenreduzierung bereitzustellen, das beim Auftragen zugleich eine seidige Textur ohne entsprechende übliche Zusätze aufweist, elastizitätsverbessernd wirkt und einen langanhaltenen Feuchthaltungseffekt aufweist.

Erfindungsgemäß besteht das Antifalten-Kosmetikum aus einem W/O-Siliconölsystem und umfasst die folgenden aktiven Bestandteile (in Gew-% und bezogen auf das Gesamtgewicht des Kosmetikums)
0,05 bis 3 % eines Extraktes von Papaver,
0,05 bis 2 % eines Extraktes von Passiflora,
0,05 bis 3 % eines Extraktes von Mentha,
0,05 bis 3 % eines Extraktes von Myrtus
neben üblichen kosmetischen Hilfsstoffen, Trägerstoffen, Wirkstoffen oder Gemischen davon, deren Anteil zu 100 % ergänzt wird.

Es wurde gefunden, dass eine Kombination der vier genannten speziellen Pflanzenextrakte eine Antifaltenwirkung auf menschlicher Haut ausübt und deutliche Verbesserungen auch bei Altershaut hinsichtlich der feinen Hautfalten erzielt werden können.

Zusätzlich zu den genannten aktiven Bestandteilen können bevorzugt 0,5 bis 15 Gew-% einer Lösung des Hexapeptids Acetyl-Glu-Glu-Met-Gln-Arg-Arg-NH₂ enthalten sein mit einem Gehalt des reinen Peptids von 0,5 g/l. Besonders bevorzugt sind 0,5-2,0 Gew-%. Die Antifaltenwirkung der erfindungsgemäßen Kombination von Pflanzenextrakten wird überraschend verstärkt durch die Zugabe des genannten Hexapeptids, das vermutlich die für die Faltenbildung gegebenenfalls verantwortliche übermäßige Catecholaminfreisetzung hemmt.

Vorzugsweise ist der aufbereitete Extrakt von Papaver ein Extrakt von P. rhoeas, P. adulis, P. incarnata, P.laurifolia, P. quadrangularis, P. somniferum oder Gemischen davon. Der Papaver-Extrakt ist ein Extrakt mit einem mehrwertigen Alkohol, wie z.B. Propylenglycol des Samens von Mohn bei 20-50 °C, gegebenenfalls im Vakuum.

Weiterhin bevorzugt ist der aufbereitete Extrakt von Mentha ein Extrakt von Mentha aquatica, Mentha arvensis, Mentha piperita, Mentha pulegium, Mentha rotundifolia, Mentha viridis oder Gemischen davon mit einem Gehalt an Mentha von etwa 0,5 Gew-%, bezogen auf das Gesamtgewicht der Extraktaufbereitung, die weiterhin Wasser, Stabilisierungs- und Konservierungsmittel enthalten kann (INCI: Water&Mentha piperita leaf extract). Die Extraktion erfolgt bei 20-50 °C.

Die Extrakte von Passiflora, z.B. P. incarnata, und von Myrtus, z.B. M. communis, M. communis 'Flore Pleno', M. communis ssp. tarentia, sind Blütenextrakte und werden mittels Propylenglycol (PPG) bei Temperaturen von 20-50 °C gewonnen. Die speziellen Gehalte im aufbereiteten Extrakt liegen bei etwa 2-3 Gew-%, bezogen auf das Gesamtgewicht der Extraktaufbereitung, die weiterhin Wasser, PPG und Konservierungsmittel enthalten kann (INCI: Water&Passiflora incarnata flower extract bzw. Water&Myrtus communis flower extract).

Der Myrtenextrakt (z.B. Myrtus communis) ist ein wässrigalkoholischer oder rein alkoholischer Extrakt von Blüten und Blättern, wobei der Alkohol ein mehrwertiger Alkohol wie Propoylenglycol ist, und die Extraktion bei 20-50 °C durchgeführt wird.

Das erfindungsgemäße Kosmetikum hat eine Textur, bei der sich die 0/W-Emulsion beim Auftragen und leichten Verreiben auf der Haut in ein Gel umwandelt und dabei ein besonderes Weichheitsgefühl hervorruft. Es tritt keinerlei Klebrigkeit auf. Insgesamt wird eine Art Botox-Effekt erzielt, der sich allerdings in einer Faltenreduzierung und -abflachung darstellt und zu einer jung aussehenden Haut führt.

Darüber hinaus ist eine Elastizitätsverbesserung der Haut nach 4-wöchiger Behandlung von bis zu 40 % gefunden worden. Das Halten von hohen Feuchtigkeitsanteilen wurde über 8 Stunden festgestellt und in einer Langzeitstudie über 4 Wochen, so dass insgesamt ein signifikanter "long lasting"-Effekt für diese Zusammensetzung der Erfindung gefunden wurde.

Insgesamt zeigte sich für das erfindungsgemäße Kosmetikum ein Hautglättungseffekt, wie er von den Einzelbestandteilen nicht zu erwarten war. Das Erreichen der seidigen Textur tritt insbesondere ohne den Zusatz der sonst üblichen Pigmente oder Puder auf, so dass diese gänzlich weggelassen werden können.

Das erfindungsgemäße Kosmetikum enthält weiterhin kosmetische Hilfs- und Trägerstoffe, wie sie üblicherweise in solchen Zubereitungen verwendet werden, z.B. Wasser, Konservierungsmittel, Farbstoffe, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Besonders bevorzugt ist der Zusatz von Antioxidationsmitteln und Radikalfängern. Zu derartigen Substanzen gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivate; sowie Gemische dieser Substanzen.

Das erfindungsgemäße Siliconölsystem besteht insbesondere aus einer Kombination von Siliconölen mit einem Silicongel. Vorteilhaft einzusetzende Siliconöle sind z.B. Cyclohexasiloxane, Cyclopentasiloxane, Cyclotetrasiloxane, Dimethicone und Gemische davon.

Der Anteil der Siliconöle liegt dabei bevorzugt im Bereich von 5 bis 15 Gew-%, bezogen auf das Gesamtgewicht des Kosmetikums.

Kombinationen dieser Siliconöle mit Silicongelen sind z.B. solche mit Dimethicone & PEG 10 Dimethicone Crosspolymer & PEG 15 Dimethicone Crosspolymer (73:13,5:13,5) oder mit Dimethicone & PEG 10 Dimethicone Crosspolymer & PEG 15 Dimethicone Crosspolymer (73:13,5:13,5).

Das erfindungsgemäße Kosmetikum kann zur Herstellung der O/W-Emulsionen Emulgatoren enthalten. Geeignete Emulgatoren für O/W-Emulsionen sind beispielsweise Anlagerungsprodukte von 2-30 Mol Ethylenoxid an lineare C₈-C₂₂-Fettalkohole, an C₁₂-C₂₂-Fettsäuren und an C₈-C₁₅-Alkylphenole; C₁₂-C₂₂-Fettsäuremono- und -diester von Anlagerungsprodukten von 1-30 Mol Ethylenoxid an Glycerin.

Wie bereits beschrieben, ist der Zusatz von Pigmenten nicht erforderlich. Gewünschtenfalls können jedoch als kosmetische Hilfsstoffe Pigmente, Pigmentgemische oder Pulver mit pigmentartiger Wirkung hinzugegeben werden, was jedoch nicht bevorzugt ist. Darunter sind auch solche mit Perlglanz-Effekt zu verstehen. Diese Pigmente oder Puder können zum Beispiel sein Siliciumdioxid, Eisenoxide, natürliche Aluminiumsilicate wie Ocker, Titan(di)oxid, Glimmer, Kaolin, manganhaltige Tone wie Umbra und roter Bolus, Calciumcarbonat, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, sowie Glimmer-Titanoxid-organischer Farbstoff.

Polyole sind ebenfalls mögliche Bestandteile des erfindungsgemäßen Kosmetikums. Dies sind z.B Propylenglycol, Dipropylenglycol, Ethylenglycol, Isoprenglycol, Glycerin, Butylenglycole, Sorbitol und Gemische davon. Der Anteil des Polyols liegt im Bereich von 0,1 bis 40 Gew-%, vorzugsweise von etwa 5% bis etwa 20 Gew-% der Gelzusammensetzung.

Ein weiterer Zusatz für das erfindungsgemäße Kosmetikum ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser. Dies ist z.B. ein Wirkstoffkomplex gemäß Beispiel 1 oder 2 von WO99/66881 oder z.B. ein Wirkstoffkomplex gemäß Beispiel 1 von WO 01/26617. Überraschenderweise führt der Zusatz dieser Wirkstoffzubereitung zu der erfindungsgemäßen Kombination zu einer deutlich verbesserten Wirksamkeit bei den radikalfangenden Eigenschaften (z.B. Radikalschutzfaktor RPF des Wirkstoffkomplexes von Beispiel 2 von WO 99/66881 = 2120; RPF der erfindungsgemäßen Extraktkombination in Beispiel 1 = 840; RPF Wirkstoffkomplex plus Extraktkombination = 3230 Radikale·10¹⁴/mg).

Darüber hinaus kann das erfindungsgemäße Kosmetikum vorteilhaft entsprechende wasser- und/oder öllösliche UVA-oder UVB-Filter oder beide enthalten. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Die Verwendung der erfindungsgemäßen kosmetischen Zusammensetzungen kann z.B. erfolgen in Form von Sonnencremes, Sonnengelen, After-sun-Produkten, Tagescremes, Nachtcremes, Masken, Körperlotionen, Reinigungsmilch, Körperpuder, Augenkosmetik, Haarmasken, Haarspülungen, Haarshampoos, Duschgelen, Duschölen, Badeölen und in Produkten der dekorativen Kosmetik wie Deo-Stiften, Parfüm-Stiften, Lippenstiften, Gelen, Lidschattens, Kompaktproduktes wie Kompaktpuder oder Kompaktwachs, Rouge, Grundierung, Make-up usw. Die Herstellung derartiger Produkte erfolgt auf eine Weise, wie sie dem Fachmann auf diesem Gebiet bekannt ist.

Besonders bevorzugt sind Cremes, Gele, Masken, Lotionen, Augenkosmetik, Make-ups, da die Auftragung dieser Anwendungsformen meist zeitlich regelmäßig erfolgt. Die dabei erzielten Wirkungen als Faltenreduzierung oder -abflachung sind beispielsweise aus den Fotos zu entnehmen. Bereits nach 7 Tagen bei täglich zweimaligem Auftragen auf die Gesichtshaut sind merkliche Verbesserungen erkennbar.

Die Erfindung soll nachstehend durch Beispiele näher erläutert werden. Alle Angaben erfolgen in Gewichtsprozent, sofern nichts anderes angegeben ist.

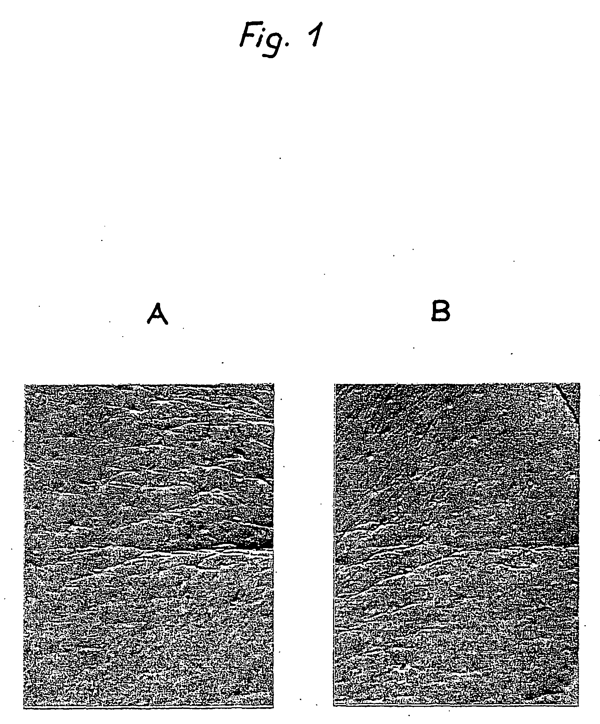

In der dazugehörigen Zeichnung zeigt
Fig. 1: Foto in vivo-behandelter Hautpartie mit Creme von Beispiel 2 (A) vor der Behandlung und (B) nach 7-tägiger Behandlung.

### Beispiel 1 Hautcreme für normale Haut

| **Phase A** | |
|---|---|
| Wasser | bis 100 % |
| Glycerin | 3,0 |
| Propylenglycol | 3,0 |

| **Phase B** | |
|---|---|
| Silicongel(Dimethicone & PEG 15 | |
| Dimethicone Crosspolymer) | 4,8 |
| Dimethicone silicone | 11,0 |

| **Phase C** | |
|---|---|
| Vitamingemisch (B₂, B₆, B₁₂, C, E, D) | 1,0 |
| Ethanol | 5,0 |
| RPF-Komplex* | 0,5 |
| Mentha-Extrakt | 2,5 |
| Passiflora-Extrakt | 0,5 |
| Papaver-Extrakt | 1,0 |
| Myrtus-Extrakt | 0,5 |
| TiO₂ | 0,5 |
| Vitamin A-palmitat | 0,05 |
| Parfüm | 0,5 |
| Konservierungsmittel | 0,5 |

| | |
|---|---|
| * Wirkstoffkomplex gemäß Beispiel 1 von WO99/66881 | |

Die Phasen A und B wurden separat hergestellt und dabei jeweils auf etwa 80 °C erhitzt. Beide Phasen wurden unter Rühren miteinander vereinigt, 20 Minuten homogenisiert. Das Gemisch wurde auf 60 °C abgekühlt, etwa 5 Minuten homogenisiert, dann auf 50 °C abgekühlt und nochmals 5 Minuten homogenisiert. Dann wurde auf 35 °C abgekühlt und die separat bei Temperaturen unter 35 °C hergestellte Phase C hinzugegeben und nochmals einige Minuten homogenisiert.

### Beispiel 1a

Der Phase C wurden 0,8 % des Hexapeptids Acetyl-Glu-Glu-Met-Gln-Arg-Arg-NH₂ zugesetzt.

### Beispiel 2 Körpercreme I

| **Phase A** | |
|---|---|
| Wasser | bis 100 % |
| Glycerin | 10,0 |
| Propylenglycol | 3,0 |

| **Phase B** | |
|---|---|
| Silicongel (Dimethicone & PEG 10 | |
| Dimethicone Crosspolymer) | 5,8 |
| Dimethicone silicone | 12,0 |

| **Phase C** | |
|---|---|
| Vitamingemisch | 1,0 |
| Alkohol | 10,0 |
| RPF-Komplex* | 0,5 |
| Mentha-Extrakt | 2,0 |
| Passiflora-Extrakt | 0,3 |
| Papaver-Extrakt | 2,0 |
| Myrtus-Extrakt | 1,5 |
| Vitamin A-palmitat | 0,5 |
| Parfüm | 0,5 |
| Konservierungsmittel | 0,5 |

| | |
|---|---|
| * Wirkstoffkomplex gemäß Beispiel 2 von WO99/66881 Es wurde wie im Beispiel 1 verfahren. | |

### Beispiel 2a

Der Phase C wurden 1,3 % des Hexapeptids zugesetzt.

### Beispiel 3 Creme für trockene Haut

| **Phase A** | |
|---|---|
| Wasser | bis 100 % |
| Glycerin | 5,0 |
| Propylenglycol | 3,0 |

| **Phase B** | |
|---|---|
| Silicongel (Dimethicone & PEG 10 & PEG 15 Dimethicone Crosspolymer) | 6,9 |
| Dimethicone silicone | 14,8 |

| **Phase C** | |
|---|---|
| Vitamingemisch | 1,0 |
| Alkohol | 8,0 |
| RPF-Komplex* | 0,8 |
| Mentha-Extrakt | 2,5 |
| Passiflora-Extrakt | 0,5 |
| Papaver-Extrakt | 1,2 |
| Myrtus-Extrakt | 0,5 |
| TiO₂/SiO₂-Pigment | 0,5 |
| Vitamin A-palmitat | 0,1 |
| Parfüm | 0,5 |
| Konservierungsmittel | 0,5 |

| | |
|---|---|
| * Wirkstoffkomplex gemäß Beispiel 1 von WO 01/26617. Es wurde wie im Beispiel 1 verfahren. | |

### Beispiel 3a

Der Phase C wurden 1,5 % des Hexapeptids zugesetzt sowie 1,1 % des RPF-Komplexes von Beispiel 2 aus WO99/66881.

### Beispiel 4 Körpercreme II

| **Phase A** | |
|---|---|
| Wasser | bis 100 % |
| Glycerin | 10,0 |
| Propylenglycol | 3,0 |

| **Phase B** | |
|---|---|
| Silicongel (Demethicone & PEG 10 & PEG 15 Dimethicone Crosspolymer) | 5,8 |
| Dimethicone silicone | 12,0 |

| **Phase C** | |
|---|---|
| Vitamingemisch | 1,0 |
| Alkohol | 10,0 |
| Hexapeptid* | 1,0 |
| Mentha-Extrakt | 2,0 |
| Passiflora-Extrakt | 0,3 |
| Papaver-Extrakt | 2,0 |
| Myrtus-Extrakt | 1,5 |
| Vitamin A-palmitat | 0,5 |
| Parfüm | 0,5 |
| Konservierungsmittel | 0,5 |

| | |
|---|---|
| *Acetyl-Glu-Glu-Met-Gln-Arg-Arg-NH₂ Es wurde wie im Beispiel 1 verfahren. | |

### Beispiel 4a

In der Phase C wurde kein Hexapeptid zugesetzt.

### Beispiel 5 Vergleichsversuch Feuchtigkeitsverbesserung

Es wurden Haut-Feuchtigkeitsmessungen mit einem Corneometer bei 18 Probandinnen mit trockener Mischhaut durchgeführt. Eingesetzt wurde ein Corneometer CM 825 (Courage&Khazaka, Deutschland) bei 22 °C und 56 % relativer Luftfeuchtigkeit. 2 Stunden nach einer vorherigen Hautreinigung wurden die unterschiedlichen Cremes aufgetragen. Die Ergebnisse der Feuchthaltung in % sind als Mittelwerte in der folgenden Tabelle aufgeführt.

**Tabelle 1**

| **Zeit** | **Creme von Bsp. 1** | | **Creme von Beispiel 2a** | | **Creme von Bsp. 1 ohne Extrakte** | |
|---|---|---|---|---|---|---|
| | **Mittelwert** | **Zunahme** | **Mittelwert** | **Zunahme** | **Mittelwert** | **Zunahme** |
| vorher | 42,5 | - | 40 | - | 42,5 | - |
| 0,5 h | 58 | +36 % | 51 | +28 % | 52 | +22 % |
| 2 h | 58 | +36 % | 53 | +33 % | 53 | +24 % |
| 6 h | 56 | +32 % | 54 | +35 % | 52 | +22 % |
| 24 h | 60 | +41 % | - | - | 45 | + 5 % |
| 2 Wochen | 62 | +45 % | 59 | +48 % | - | - |
| 4 Wochen | 65 | +53 % | 63 | +54 % | - | - |

Der Vergleich zeigt eine deutlich bessere Feuchtigkeitshaltung der Creme von Beispiel 1 der vorliegenden Erfindung mit dem Komplex gegenüber einer Creme ohne diesen Komplex. Die Vergleichscreme ohne den Komplex zeigte zwar nach 8 Stunden noch einen erhöhten Feuchtigkeitswert, fiel jedoch nach 24 h auf nahe null ab, während die erfindungsgemäße Creme erhöhte Feuchtigkeitswerte von +41 % hatte. Besonders signifikant ist die Beibehaltung und Steigerung dieser Werte über einen Zeitraum von 4 Wochen.

### Beispiel 6 Vergleichsversuche Antifaltenwirkung

Es wurden Tests durchgeführt zum Nachweis der Antifaltenwirkung. Der Test wurde bei 21 Probanden/Probandinnen im Alter von 42 bis 61 Jahren durchgeführt. Das Mikrorelief von Abschnitten der Gesichtshaut (Augenpartie, Mundwinkel, Nasenpartie) wurde mittels einer Silikonmasse abgenommen, die Masse ausgehärtet und das erhaltene Negativrelief elektrooptisch vermessen in Bezug auf Höhe und Anzahl der Falten. Unmittelbar nach der Abnahme des Mikroreliefs erfolgte das erste Auftragen einer Creme im Gesicht der Probanden und danach täglich zweimal eine Wiederholung des Auftragens mit einer Menge von etwa 2mg/cm².
- Gruppe 1:: 12 Probanden erhielten die Creme von Beispiel 1;
- Gruppe 2:: 6 Probanden erhielten die Creme von Beispiel 2a;
- Gruppe 3:: 3 Probanden erhielten eine Creme, die allein aus der Basisformulierung von Beispiel 1 ohne Wirkstoffe bestand (Placebo).

Kontrollmessungen durch Abnehmen des Mikroreliefs der gleichen Hautpartien der einzelnen Probanden wurden am 14. und 28. Tag nach der ersten Messung vorgenommen. In dieser Zeit waren alle Probanden keinen besonderen Belastungen durch Sonneneinstrahlung unterworfen. Die ermittelten Werte sind statistische Mittelwerte über einen ausgewählten Bereich des Mikroreliefs.

**Tabelle 2**

| | Anzahl der Probanden | | |
|---|---|---|---|
| | Gruppe 1 | Gruppe 2 | Gruppe 3 |
| Verbesserung der Faltentiefe nach 14 Tagen | | | |
| um 10 - 30 % | 8 | 4 | 1 |
| Um 30 - 50 % | 4 | - | - |

| Verbesserung der Faltentiefe nach 28 Tagen | | | |
|---|---|---|---|
| um 10 - 30 % | 2 | 1 | 1 |
| um 40 - 50 % | 7 | 3 | - |
| > 50 % | 2 | 2 | - |

Die Gruppe 1 zeigt eine sehr gute Reduzierung der Faltentiefe nach 28 Tagen bei 75 % der Probanden. Gegenüber Gruppe 1 zeigt die erfindungsgemäße Zusammensetzung gemäß Beispiel 2a mit der Gruppe 2 noch einmal eine signifikante Verbesserung, die auf die Wechselwirkung mit dem Hexapeptid zurückzuführen ist.

## Patentansprüche

1. Antifalten-Kosmetikum, **dadurch gekennzeichnet, daß** es in einem W/O-Siliconölsystem folgende Bestandteile umfaßt
0,05 bis 3,0 eines Extraktes von Papaver,
0,05 bis 2,0 eines Extraktes von Passiflora,
0,05 bis 3,0 eines Extraktes von Mentha,
0,05 bis 3,0 eines Extraktes von Myrtus,
neben üblichen kosmetischen Hilfsstoffen, Trägerstoffen, Wirkstoffen oder Gemischen davon, deren Anteil zu 100 % ergänzt wird.

2. Antifalten-Kosmetikum nach Anspruch 1, **dadurch gekennzeichnet, daß** es als weiteren Wirkstoff 0,5 bis 15 Gew-% einer Lösung des Hexapeptids Acetyl-Glu-Glu-Met-Gln-Arg-Arg-NH₂ enthält, dessen Gehalt des reinen Peptids 0,5 g/l beträgt.

3. Antifalten-Kosmetikum nach Anspruch 1, **dadurch gekennzeichnet, daß** der Extrakt von Papaver ein Extrakt der Samen von P. adulis, P. rhoeas, P. incarnata, P.laurifolia, P. quadrangularis, P. somniferum oder Gemischen davon ist.

4. Antifalten-Kosmetikum nach Anspruch 1, **dadurch gekennzeichnet, daß** der Extrakt von Mentha ein Extrakt von M. aquatica, M. arvensis, M. piperita, M. pulegium, M. rotundifolia, M. viridis oder Gemischen davon ist.

5. Antifalten-Kosmetikum nach Anspruch 1, **dadurch gekennzeichnet, daß** die Siliconöle des Siliconölsystems ausgewählt sind aus der Gruppe, bestehend aus Cyclohexasiloxane, Cyclopentasiloxane, Cyclotetrasiloxane, Dimethicone und Gemischen davon.

6. Antifalten-Kosmetikum nach Anspruch 1, **dadurch gekennzeichnet, daß** als Silicongel Dimethicone & PEG 10 Dimethicone Crosspolymer & PEG 15 Dimethicone Crosspolymer zusammen mit einem oder mehreren Siliconölen enthalten ist.

7. Antifalten-Kosmetikum nach Anspruch 1, **dadurch gekennzeichnet, daß** es als weiteren Wirkstoff 0,2 bis 1,5 Gew-% eines Wirkstoffkomplexes enthält, bezogen auf das Gesamtgewicht des Kosmetikums, bestehend aus 0,1-10 Gew-% Rindenextrakt von Quebracho blanco, 0,1-10 Gew-% Seidenraupenextrakt, 0,1-5 Gew-% eines Hydrogels, 0,1-30 Gew-% Phospholipide und 45-99,6 Gew-Wasser, bezogen auf das Gesamtgewicht des Wirkstoffkomplexes.

8. Antifalten-Kosmetikum nach Anspruch 1, **dadurch gekennzeichnet, daß** das Kosmetikum keine Pigmente oder Puder enthält.

9. Antifalten-Kosmetikum nach Anspruch 1, **dadurch gekennzeichnet, daß** es jeweils in einer kosmetischen Zusammensetzung umfasst 0,8-2,4 eines Extraktes von Papaver, 0,1-0,9 eines Extraktes von Passiflora, 1,2-2,6 eines Extraktes von Mentha, 0,4 bis 2,8 eines Extraktes von Myrtus und 0,5-2,0 einer Lösung des Hexapeptids Acetyl-Glu-Glu-Met-Gln-Arg-Arg-NH₂.

## Claims

1. An anti-wrinkle cosmetic comprising the following ingredients in a W/O silicone oil system
0.05 to 3.0 % by weight of an extract from Papaver,
0.05 to 2.0 % by weight of an extract from Passiflora,
0.05 to 3.0 % by weight of an extract from Mentha,
0.05 to 3.0 % by weight of an extract from Myrtus,
in addition to usual cosmetic auxiliaries, carriers, active agents or mixtures thereof, which make up the remainder up to 100 %.

2. An anti-wrinkle cosmetic according to claim 1, wherein said cosmetic contains 0.5 to 15 % by weight of a solution of the hexapeptide acetyl-Glu-Glu-Met-Gln-Arg-Arg-NH₂ as an additional active agent, the concentration of the pure peptide in said solution being 0.5 g/l.

3. An anti-wrinkle cosmetic according to claim 1, wherein said extract from Papaver is an extract from the seeds of P. adulis, P. rhoeas, P. incarnata, P. laurifolia, P. quadrangularis, P. somniferum or mixtures thereof.

4. An anti-wrinkle cosmetic according to claim 1, wherein said extract from Mentha is an extract from M. aquatica, M. arvensis, M. piperita, M. pulegium, M. rotundifolia, M. viridis or mixtures thereof.

5. An anti-wrinkle cosmetic according to claim 1, wherein the silicone oils of said silicone oil system are selected from the group consisting of cyclohexasiloxane, cyclopentasiloxane, cyclotetrasiloxane, dimethicone and mixtures thereof.

6. An anti-wrinkle cosmetic according to claim 1, wherein said cosmetic contains Dimethicone & PEG 10 Dimethicone Crosspolymer & PEG 15 Dimethicone Crosspolymer as silicone gel, together with one or several silicone oil (s).

7. An anti-wrinkle cosmetic according to claim 1, wherein said cosmetic contains 0.2 to 1.5 % by weight of an active complex, relative to the cosmetic's total weight, as an additional active agent, which complex consists of 0.1-10 % by weight of an extract from the bark of Quebracho blanco, 0.1-10 % by weight of a silkworm extract, 0.1-5 % by weight of a hydrogel, 0.1-30 % by weight phospholipids and 45-99.6 % by weight water relative to the total weight of the active complex.

8. An anti-wrinkle cosmetic according to claim 1, wherein said cosmetic contains neither pigments nor powder.

9. An anti-wrinkle cosmetic according to claim 1, wherein said cosmetic comprises 0.8-2.4 % by weight of an extract from Papaver, 0.1-0.9 % by weight of an extract from Passiflora, 1.2-2.6 % by weight of an extract from Mentha, 0.4-2.8 % by weight of an extract from Myrtus and 0.5-2.0 % by weight of a solution of the hexapeptide acetyl-Glu-Glu-Met-Gln-Arg-Arg-NH₂ in a cosmetic composition.

## Revendications

1. Produit cosmétique antirides, **caractérisé en ce qu'**il comprend les constituants suivants dans un système E/H d'huile de silicone
0,05 à 3,0 d'un extrait de pavot,
0,05 à 2,0 d'un extrait de passiflore,
0,05 à 3,0 d'un extrait de menthe,
0,05 à 3,0 d'un extrait de myrte,
en plus des auxiliaires, supports, agents actifs cosmétiques usuels ou leurs mélanges, dont la quantité est complétée pour faire 100%.

2. Produit cosmétique antirides selon la revendication 1, **caractérisé en ce qu'**il contient comme autre agent actif, 0,5 à 15% en poids d'une solution de l'hexapeptide acétyl-Glu-Glu-Met-Gln-Arg-Arg-NH₂, dont la teneur en peptide pur s'élève à 0,5 g/litre.

3. Produit cosmétique antirides selon la revendication 1, **caractérisé en ce que** l'extrait de pavot est un extrait de graines de *P. adulis*, de *P. rhoeas,* de *P. incarnata,* de *P. laurifolia,* de *P. quadrangularis,* de *P. somniferum* ou de leurs mélanges.

4. Produit cosmétique antirides selon la revendication 1, **caractérisé en ce que** l'extrait de menthe est un extrait de *M. aquatica, M. arvensis, M. piperita, M. pulegium, M. rotundifolia, M. viridis* ou leurs mélanges.

5. Produit cosmétique antirides selon la revendication 1, **caractérisé en ce que** les huiles de silicone du système d'huile de silicone sont choisies parmi le groupe consistant en le cyclohexasiloxane, le cyclopentasiloxane, le cyclotétrasiloxane, la diméthicone et leurs mélanges.

6. Produit cosmétique antirides selon la revendication 1, **caractérisé en ce qu'**il contient comme gel de silicone, la diméthicone & le PEG 10 Dimethicone Crosspolymer & le PEG 15 Dimethicone Crosspolymer, avec une ou plusieurs autres huiles de silicone.

7. Produit cosmétique antirides selon la revendication 1, **caractérisé en ce qu'**il contient comme autre agent actif, 0,2 à 1,5% en poids d'un complexe d'agents actifs, sur base du poids total du produit cosmétique, consistant en 0,1-10% en poids d'extrait d'écorce de *Quebracho blanco,* 0,1-10% en poids d'extrait de ver à soie, 0,1-5% en poids d' un hydrogel, 0,1-30% en poids de phospholipides et 45-99,6% en poids d'eau, sur base su poids total du complexe d'agents actifs.

8. Produit cosmétique antirides selon la revendication 1, **caractérisé en ce que** le produit cosmétique ne contient aucun pigment ou poudre.

9. Produit cosmétique antirides selon la revendication 1, **caractérisé en ce qu'**il comprend, chaque fois dans une préparation cosmétique, 0,8-2,4 d'extrait de pavot, 0,1-0,9 d'un extrait de passiflore, 1,2-2,6 d'un extrait de menthe, 0,4 à 2,8 d'un extrait de myrte et 0,5-2,0 d'une solution de l'hexapeptide acétyl-Glu-Glu-Met-Gln-Arg-Arg-NH₂.
